# EUROPEAN PATENT APPLICATION

(11) **EP 3 431 093 A1**
(43) Date of publication of application: **23.01.2019**
(21) Application number: 18183989.5
(22) Date of filing: 17.07.2018
(51) Int. Cl.: A61K 31/715, A61P 1/00, A61K 35/741, A61K 45/06

(54) **WHEAT-DERIVED POLYSACCHARIDES FOR REDUCTION OF ANTIBIOTIC RESISTANCE**

(30) Priority: 17.07.2017 EP 17181623
(71) Applicant: BioActor B.V., 6229 EV Maastricht (NL)
(72) Inventor: VAN DER SAAG, Antonie Johannes, 6229 EV Maastricht (NL); POSSEMIERS, Sam, 6229 EV Maastricht (NL)
(74) Representative: LC Patents

(57) **Abstract**

The present invention is directed to a composition comprising wheat polysaccharides for use in reducing the presence and/or activity of antibiotic resistant bacteria in a subject in need thereof. The invention further provides a pharmaceutical composition, a food or feed supplement or a combination of at least two compositions for use in reducing the presence and/or activity of antibiotic resistant bacteria in a subject in need thereof. The compositions optionally comprise for example probiotics or antibiotics.

## Description

### FIELD OF THE INVENTION

The present invention is directed to a composition comprising wheat-derived arabinoxylans for use in reducing the presence and/or activity of antibiotic resistant bacteria in a subject in need thereof. The invention further provides a pharmaceutical composition, a food supplement or a combination of at least two compositions for use in reducing the presence and/or activity of antibiotic resistant bacteria in a subject in need thereof. The compositions optionally comprise for example probiotics or antibiotics.

### BACKGROUND TO THE INVENTION

Antibiotic resistance is a global public health problem. Although being a major therapeutic strategy to address numerous infectious diseases both in human and veterinary medicine, the indiscriminate and improper use of antibiotics has led to decreased susceptibility and increased resistance rates observed not only in disease-causing microbes or bacteria but also in the commensal gastro-intestinal microbiota. In the human clinical setting, antibiotic-resistant pathogens have caused numerous treatment failures that eventually led to both hospital morbidities and mortalities. Overall, the prevalence of antibiotic resistance has now become a global health problem that needs urgent attention.

The use of antibiotics has not only a tremendous impact on the pathogenic bacteria but also on the composition and functionality of the human commensal gastro-intestinal microbiota. Studies showed that intake of antibiotics results in a dramatic loss in diversity as well as in representation of specific taxa of the human microbiota, increase of antibiotic resistant strains, and up-regulation of antibiotic resistance genes. Extensive antibiotic use has pushed microbes to adapt and survive by acquiring antibiotic resistance genes that led to antibiotic-resistant strains. Such strains comprise pathogens which cause gastro-intestinal symptoms such as diarrhoea and/or constipation.

Probiotic bacteria have been observed to reduce the risk of certain types of diarrhea, constipation and respiratory infection. Concomitant use of probiotics with antibiotics has further been observed to reduce the incidence, duration and/or severity of antibiotic-associated diarrhea, although a direct link between probiotics and the development of antibiotic resistance was not shown.

The human gut ecosystem consists of a variety of different habitats and metabolic niches that are colonized by the so-called microbiota that contain more than 10¹¹ micro-organisms per gram wet weight of contents, predominantly anaerobes. The intestinal microbiota have both beneficial and pathogenic potential. The microbial community can provide protection against pathogenic bacteria, stimulates cell-mediated and humoral immune responses, and indirectly supports digestive processes by microbial fermentation. It also includes potential pathogenic organisms such as certain species of Clostridium, Escherichia, Salmonella, Shigella and Pseudomonas, as well as yeasts such as *Candida albicans.* Consumption of non-digestible food ingredients such as inulin-type fructans, known as prebiotics, favor the growth and activity of certain colonic bacteria, such as bifidobacteria and lactobacilli, generally regarded as beneficial to the host. Current antibiotics in general indiscriminatorily affect pathogenic and beneficial bacteria. There is a continuing need for products that suppress pathogenic bacteria, especially antibiotic-resistant bacteria, while maintaining or even stimulating growth and activity of beneficial bacteria.

In addition, patients and consumers are currently strongly focused on the consumption of natural products and there is a general aversion against synthetically created drugs. Therefore, there is also a continuing need for naturally-derived products for use in reducing antibiotic-resistant bacteria.

### SUMMARY OF THE INVENTION

The present invention surprisingly showed that specific polysaccharides, in particular certain wheat-derived arabinoxylans (AX) reduce the presence and/or activity of antibiotic resistant bacteria.
Arabinoxylans (AX), the main non-starch polysaccharide of cereal grains, were previously shown to exert a beneficial effect on the organization of the intestinal microbial community in the lumen and in particular at the site of the gut mucosa, thereby exerting prebiotic effects, in particular by modulating the mucosa-associated microbial community towards a relative increase of health beneficial bacteria, such as bifido bacteria and lactobacilli (see e.g. WO2010020639). Though, so far, no suggestion was ever made towards an activity on antibiotic resistance and/or an activity towards reducing gastro-intestinal symptoms such as diarrhoea, constipation and/or gastro-intestinal discomfort. Therefore, the present invention provides products that are able to suppress the presence and/or activity antibiotic resistant bacteria, while maintaining and even stimulating the growth of beneficial bacteria, such as bifidobacteria.

Therefore, the present invention provides a composition comprising wheat-derived arabinoxylans for use in reducing antibiotic resistance, more specific for use in reducing the presence and/or activity of antibiotic resistant bacteria in a subject in need of such reduction. In a particular embodiment, the present invention provides compositions for affecting antibiotic resistant bacteria in the gastro-intestinal tract, in particular in the gut. The invention therefore provides a composition comprising at least 30% (w/w) of wheat-derived arabinoxylans with an average degree of polymerisation of at least 50 for use in reducing the presence and/or activity of antibiotic resistant bacteria in a subject in need thereof. In a further embodiment, the composition for use according to the present invention comprises at least 30% (w/w) of wheat-derived arabinoxylans with an average degree of polymerisation between 50 and 150 and an average degree of substitution between 0.6 and 0.8. In yet a further embodiment, said composition for use according to the present invention comprises a protein content of up to 25% (w/w).
As already indicated herein above, the present invention provides a composition comprising at least 30% (w/w) of wheat derived arabinoxylans with an average degree of polymerisation of at least 50 for use in reducing the presence and/or activity of antibiotic resistant bacteria in a subject in need thereof. In a further preferred embodiment, said composition comprises at least 50% (w/w) wheat derived arabinoxylans; in an even further embodiment, said composition comprises at least 55% (w/w) wheat derived arabinoxylans. Typical for the present invention is further that the wheat-derived arabinoxylans are preferably water-soluble arabinoxylans.

In further embodiments of the present invention, the composition comprising wheat-derived arabinoxylans for use in reducing the presence and/or activity of antibiotic resistant bacteria, may further be characterized in having one or more of the following characteristics:
- comprising at least 30% (w/w) of arabinoxylans; in particular at least 50% (w/w) of arabinoxylans; more in particular at least 60% (w/w) of arabinoxylans; in particular about 50% - 85% (w/w) of arabinoxylans; even more in particular 55% - 85% (w/w) arabinoxylans.
- having an average degree of polymerisation of at least 50; in particular an average degree of polymerisation between 50 and 150.
- having an average degree of substitution between 0.4 and 0.9; in particular between 0.6 and 0.8; in particular at least 0.5.
- comprising a protein content of up to 5%, 10%, 15%, 20%, 22% or 25% (w/w); in particular a protein content of up to about 2% (w/w).
In another embodiment, the arabinoxylan preparations according to the different embodiments of the invention are mainly derived from the wheat endosperm fraction.

In a particular embodiment, the arabinoxylan preparations according to the different embodiments of the invention have a total sugar concentration of at least 50% (w/w); in particular about and between 50-85% (w/w); in particular having a sugar content of about 56 % (w/w); wherein the sugar content typically comprises;
- about 10 to 40% (w/w) of arabinose; more in particular about 15 to 30% (w/w); even more in particular about 25% of arabinose;
- about 15 to 55% (w/w) of xylose; more in particular about 20 to 30% (w/w); even more in particular about 35% of xylose;
- about 0 - 15% (w/w) of galactose; in particular about 9% of galactose;
- about 1 - 15% (w/w) of glucose; in particular about 8% of glucose.

In another aspect of the present invention, the composition comprising at least 30% of wheat-derived arabinoxylans with an average degree of polymerisation of at least 50 is for use in reducing the expression of antibiotic resistance genes in the gut microbiota. Said antibiotic resistance genes are in particular at least one gene selected from the group consisting of aminoglycoside resistance genes, beta-lactamases from Class 1, Class B, Class C, or Class D, fluoroquinolone resistance genes, and macrolide-lincosamide-streptogramine resistance gene mecA. In a further embodiment, said composition comprises wheat-derived arabinoxylans with an average degree of polymerisation between 50 and 150 and an average degree of substitution between 0.6 and 0.8. In still another embodiment, said composition for use in reducing the expression of antibiotic resistance genes comprises a protein content of up to 25% (w/w).

In another embodiment, the present invention relates to a pharmaceutical composition for use in reducing the presence and/or activity of antibiotic resistant bacteria in a subject in need thereof. Said pharmaceutical composition comprises a composition according to the different embodiments of the present invention as outliner herein above and at least one acceptable pharmaceutical carrier. In particular, said pharmaceutical composition comprises a composition comprising at least 30% (w/w) wheat-derived arabinoxylans with an average degree of polymerisation of at least 50 and at least one pharmaceutically acceptable carrier for use in reducing the presence and/or absence of antibiotic resistant bacteria in a subject in need thereof. More preferably, said pharmaceutical composition comprises a composition comprising wheat-derived arabinoxylans with an average degree of polymerisation between 50 and 150 and an average degree of substitution between 0.6 and 0.8. In a further embodiment, the pharmaceutical composition for use according to the present invention comprises a composition comprising at least 30% (w/w) of wheat-derived arabinoxylans with an average degree of polymerisation of at least 50 and a protein content of up to 25% (w/w). In another preferred embodiment, the pharmaceutical composition for use according to the present invention comprises a composition comprising at least 50% (w/w) of wheat-derived arabinoxylans. In still another embodiment, the wheat-derived arabinoxylans present in the pharmaceutical composition for use according to the different embodiments of the present invention, are water-soluble arabinoxylans.

The present invention also provides a food or feed supplement for use in reducing the presence and/or activity of antibiotic resistant bacteria in a subject in need thereof. Said food or feed supplement comprises a composition for use according to the different embodiments of the present invention. In particular, said food or feed supplement therefore comprises a composition comprising at least 30% (w/w) of wheat-derived arabinoxylans with an average degree of polymerisation of at least 50. More preferably, said food or feed supplement comprises a composition comprising at least 30% (w/w) wheat-derived arabinoxylans with an average degree of polymerisation between 50 and 150 and an average degree of substitution between 0.6 and 0.8. In yet another embodiment, the food or feed supplement comprises a composition comprising at least 30% (w/w) of wheat-derived arabinoxylans with an average degree of polymerisation of at least 50 wherein the wheat-derived arabinoxylans are water-soluble arabinoxylans.

The present invention is further directed to a combination of at least two compositions for use in reducing the presence and/or activity of resistant bacteria in a subject in need thereof. The first composition of said combination comprises a composition for use according to the different embodiments of the present invention, in particular a composition comprising at least 30% (w/w) of wheat-derived arabinoxylans with an average degree of polymerisation of at least 50 according to the different embodiments of the present invention, a pharmaceutical composition or a food or feed supplement according to the different embodiments of the present invention. The second composition of the combination can be a probiotic, for example a Bifidobacterium, an antibiotic, or any other composition. The at least two compositions of the combination according to the different embodiments of the invention can be administered simultaneously or separately.

As outlined herein above, the different compositions, food or feed supplements and/or combinations of compositions are for use in reducing the presence and/or activity of antibiotic resistance bacteria in a subject in need thereof. As used herein, a subject refers to a non-human animal or a human. In a particular embodiment, said subject is a mammal or a bird. In a further embodiment, said subject is selected from the group consisting of humans; farm animals selected from the group consisting of pigs, sheep, cattle, horses and poultry; and pet animals selected from dogs and cats. In a preferred embodiment, said subject is a human. As also indicated above, the present invention is directed to a food or feed supplement for use in reducing the presence and/or activity of antibiotic resistant bacteria in a subject in need thereof. In a particular embodiment, the present invention is directed to a food supplement for use in reducing the presence and/or activity of antibiotic resistant bacteria in humans in need thereof. In yet another embodiment, the present invention discloses a feed supplement for use in reducing the presence and/or activity of antibiotic resistant bacteria in animals, such as farm animals selected from the group comprising pigs, sheep, cattle, horses or poultry, or pet animals selected from the group comprising cats or dogs.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

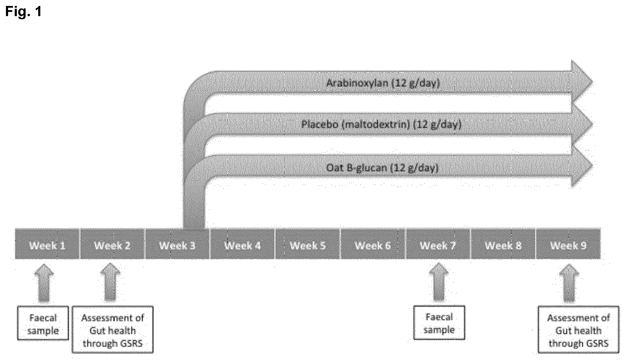

**Fig. 1** Study outline of the parallel controlled trial with 3 groups of 20 elderly people each ≥65 years and randomized to take 12g of either of the prebiotic fibers, AX-COMPOSITION (Arabinoxylan), OatWell® (Oat β-glucan) or placebo (maltodextrin) daily for 6 weeks (42 days).

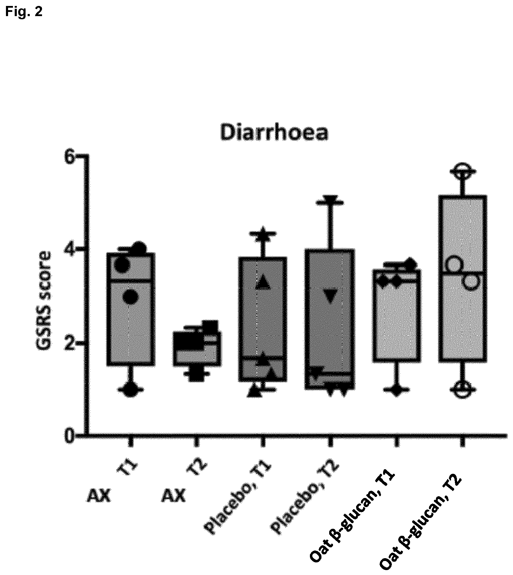

**Fig. 2** Symptoms of diarrhoea in the different intervention groups (placebo, oat-well or AX-composition). Three groups of 20 elderly people (each >65 years) were randomized to take 12 g of ether the prebiotic fibers AX-composition, Oatwell (oat beta-glucan) or placebo (maltodextrin) daily for 6 weeks, and symptoms of diarrhoea were monitored as assessed by GSRS clinical rating scale.

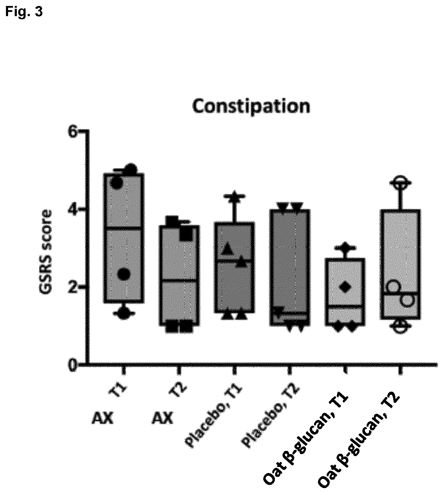

**Fig. 3** Symptoms of constipation in the different intervention groups (placebo, oat-well or AX-composition). Three groups of 20 elderly people (each >65 years) were randomized to take 12 g of ether the prebiotic fibers AX-composition, Oatwell (oat beta-glucan) or placebo (matlodextrin) daily for 6 weeks, and symptoms of constipation were monitored as assessed by GSRS clinical rating scale.

### DETAILED DESCRIPTION OF THE INVENTION

This invention relates to a composition comprising wheat-derived arabinoxylans (AX) for use in reducing the presence and/or activity of antibiotic resistant bacteria in a subject in need thereof. Further, the invention also discloses a pharmaceutical composition or food supplement comprising said composition comprising wheat-derived arabinoxylans for use in reducing the presence and/or activity of antibiotic resistant bacteria in a subject in need thereof. In a more specific embodiment of the present invention, a composition comprising at least 30% of wheat-derived arabinoxylans with an average degree of polymerisation of at least 50 is provided for use in reducing the presence and/or activity of antibiotic resistant bacteria in a subject in need thereof. Preferably, the compositions and methods of the invention are directed to reducing the presence and/or activity of antibiotic resistant bacteria; in particular to reducing the presence of antibiotic resistant bacteria.

Preferably, the AX in the composition is defined by an average degree of polymerization between 50 and 150, in particular between 50 and 100, more in particular between 60 and 80. In another preferred embodiment, the average degree of substitution of the AX used in the invention is between 0.6 and 0.8, preferably between 0.65 and 0.75. The composition for use according to the present invention is further characterized in that it preferably comprises a protein content of up to 25% (w/w). In another embodiment, the wheat-derived arabinoxylans present in the composition according to the invention, are mainly water-soluble arabinoxylans. In a further embodiment, the compositions of the invention comprise at least 30% (w/w), particularly at least 50% (w/w) of water soluble arabinoxylan, wherein the water soluble arabinoxylans are characterized by an average degree of polymerization and/or substitution as defined for arabinoxylans in general.

The dietary fiber constituents arabinoxylans (AX) are the main non-starch polysaccharide of cereal grains. These complex carbohydrates occur in cell walls of the starchy endosperm cells and the aleurone layer in most cereals (60-70% w/w) of the total carbohydrate). They can be found in the endosperm cell walls of barley (20% (w/w)) and rice (40% (w/w)). Non-endospermic tissues of wheat, particularly the pericarp and testa, also contain a very high concentration of AX (64% (w/w)). AX consist of β-(1,4)-linked D-xylopyranosyl residues to which α-L-arabinofuranose units are linked as side chains. Some arabinoses can be substituted with ferulic acid. AX can be characterized by its average degree of substitution and average degree of polymerization. The average degree of substitution refers to the average amount of arabinose moieties on the xylose backbone and is further also described as A/X ratio. It can be calculated by dividing the number of arabinose moieties by the number of xylose moieties and, thus, reflects the average number of arabinose groups per xylose group. The substitution and distribution of side chains are important factors in the physicochemical properties of AX. As for other polymers, also the average degree of polymerization (DP), i.e. the molecular weight ratio of the polymer vis-a-vis the molecular weight of the repeating units, is an important factor in the physicochemical properties of AX. As used herein, the average degree of substitution and average degree of polymerization are preferably determined by gas-liquid chromatography. For example according to the methods described in Courtin et al. (J. Chromatograph. A866 (2000) 97-104).

Percentages as used throughout the present application refer to average weight percentages unless otherwise specified or dictated by the context. Methods for the preparation of arabinoxylan compositions useful in the present invention are e.g. disclosed in WO2010020639 (A1), which is hereby incorporated by reference.

AX are present in water-extractable form in grains and in a water-unextractable fraction present in the cell wall material. Whereas the latter one needs to be extracted from wheat using for instance alkali treatment, the water-extractable fraction is readily available in the water waste streams from the wheat processing. In the current state of the art, AX are extracted by using enzymes (i.e. hemicellulases and endoxylanases) which leads to (partial) hydrolysis of AX and results in a mixture of soluble and non-soluble AX molecules with low molecular weight (WO199402874, WO2006027, WO2006002495, US6558930). The reason for the focus on short chain - hydrolyzed- AX (also called arabinoxylan oligosaccharides or AXOS) and the use of hemicellulases is that the yield of soluble long-chain AX is supposed to be too low. One reason for this low yield is the fact that endogenous enzymes are likely to degrade long-chain AX. Another reason is that the inherent viscosity of long-chain AX poses challenges for an efficient and cost effective extraction. It is also generally assumed that long-chain AX are badly water soluble because of their viscous nature. Finally, it has long be assumed that only the short chain AX (soluble and non-soluble, especially those having an average degree of polymerization of less than about 15) have interesting physiological effect.

Non-digestable oligosaccharides (NDOs) like arabinoxylans, resist digestion and absorption in the human small intestine with complete or partial fermentation in the large intestine. These carbohydrates help to maintain regularity of colonic functions and could possibly contribute to human health by reducing the risk of chronic diseases. A lot of NDOs are considered to be prebiotics. Certain short chain arabinoxylans are also known to improve the growth of beneficial bacteria in the colon, and certain long-chain water-soluble arabinoxylans have a beneficial effect on the organization of the intestinal microbial community by modulating the barrier function of the intestinal surface.

Prebiotics are non-digestible food ingredients that beneficially affect the host by selectively stimulating the growth and/or activity of one or a limited number of beneficial bacteria in the colon, thereby improving the host health. Prebiotic effects in the gut can be evaluated on the basis of the growth of health promoting bacteria such as lactobacilli and bifidobacteria, the decrease in intestinal pathogens and the increase or decrease in production of health related bacterial metbolites. The latter include for instance short chain fatty acids, which are generally believed to be positive for colonic health, but also polyamones and ammonia, which are regarded as a risk factor for colon carcinogenesis.

Based on the observation that the arabinoxylan preparations as disclosed in the present invention reduce the expression of antibiotic resistant genes, said preparations are particularly useful to reduce the presence and/or activity of antibiotic resistant bacteria in a subject. Therefore, it is to be expected that the arabinoxylan preparations for use according to the different embodiments of the present invention will have a beneficial effect on any indication related to antibiotic resistance, in particular antibiotic resistance related to gastro-intestinal infections. For example, the arabinoxylan preparations according to the present invention are beneficial for reducing gastro-intestinal complaints such as diarrhea, constipation and/or gastro-intestinal discomfort caused by the presence and/or activity of antibiotic resistant bacteria.

The effective amount of a composition according to the present invention, which is required to achieve a therapeutic effect will, of course, vary with the route of administration, the age and condition of the recipient, and the particular disorder or disease being treated. The skilled person is well-aware on how to obtain therapeutically effective amounts based on the disclosures herein. For a typical treatment a daily dosage of about 1,0 to 20,0 g; in particular of about 2,0 to 10,0 g; more in particular 5,0 g of the arabinoxylan preparations (supra) should be applied. For a typical person of 60-70 kg, this would require an intake of about 20 - 400 mg/kg per day of the arabinoxylan preparations (supra); in particular 40 - 100 mg/kg per day and more in particular 80 mg/kg per day.
Therefore, the compositions according to the invention preferably comprise between and about 0,5 g to 10,0 g of the arabinoxylan preparations as defined hereinbefore; in particular comprising between and about 1,0 g to 5,0 g of the arabinoxylan preparations per single unit dosage form.

As another aspect, the present invention provides a combination of at least two compositions, wherein the first composition is as defined herein, and the other composition(s) is another agent for modulating or improving the intestinal microbial composition and/or the barrier, hormonal or immune function of the intestinal surface, and/or for modulating or improving metabolic homeostasis, satiety, weight management, and/or for preventing the onset of cardiovascular diseases, all as described above. Examples of such other agents are; (1) probiotics, such as for example selected from the non-limiting group consisting of Bifidobacterium, Lactobacillus, Streptococcus, Enterococcus, Eubacterium, Clostridium or Saccharomyces, (ii) prebiotics, from the non-limiting group consisting of inulin, fructo-oligosaccharides or galactooligosaccharides; or (iii) antibiotics. Preferably, the compositions for use according to the present invention are combined with probiotics or antibiotics. A preferred probiotic for combination with or in the compositions of the invention is a bifidobacterium, such as *Bifidobacterium longum.*

The present invention thus relates to a composition for use according to the different embodiments of the invention. These compositions can be prepared in any known or otherwise effective dosage or product form suitable for use in providing topical or systemic delivery of the arabinoxylan preparations, which would include both pharmaceutical dosage forms as well as nutritional product forms suitable for use in the method described herein.
In a particular embodiment, the use of the products of the invention comprises oral administration. The compositions are preferably administered as oral dosage forms. Preferred dosage or product forms in this respect include oral tablets; capsules, including encapsulation in microcapsules or liposomes such as for example described in US 5827531; instant formulas, and oral liquids.

In another particular embodiment, the present invention provides a method for reducing the activity and/or presence of antibiotic resistant bacteria in a subject, the method comprising administering a composition as described herein to the subject. In a further embodiment, the present invention provides a method for reducing the activity and/or presence of antibiotic resistant bacteria the gastro-intestinal tract of a subject, in particular the gut of a subject, the method comprising administering a composition, a food or feed supplement, or a combination of compositions of the invention to the subject. In an even further embodiment, the method comprises orally administering a composition of the invention to the subject.

As described herein before, the compositions of the invention can be used to reduce the presence and/or activity of antibiotic resistant bacteria. In a particular embodiment, the compositions of the invention are used to reduce the presence of antibiotic resistant bacteria. In a further embodiment, the compositions of the invention are used to simultaneously reduce the presence of antibiotic resistant bacteria and increase the presence of beneficial bacteria, such as lactobacilli and bifidobacteria, in particular bifidobacteria.

In another embodiment, the compositions of the present invention can be used to reduce gastro-intestinal complaints that are associated with the presence of antibiotic resistant bacteria. These gastro-intestinal complaints are selected from the group comprising diarrhea, constipation and/or gastro-intestinal discomfort. In a preferred embodiment, the compositions of the present invention are used for reducing gastro-intestinal complaints associated with antibiotic resistant bacteria; particularly caused by antibiotic resistant bacteria.

The present invention is also directed to a pharmaceutical composition comprising a composition for use according to the different embodiments of the invention and a pharmaceutically acceptable carrier. The pharmaceutical compositions of the present invention can be prepared by any known or otherwise effective method for formulating or manufacturing the selected product form. Methods for preparing the pharmaceutical compositions according to the present invention can be found in "Remington's Pharmaceutical Sciences"; Mid. Publishing Co., Easton, Pa., USA.

In yet another embodiment, the present invention provides a food, nutritional or feed supplement comprising a composition for use according to the different embodiments of the invention. Generally, a food or feed supplement according to the present invention comes as any form described in the present description, and in particular fat-based food products (butter, oil, margarine), bread, cookies, or oil kept food products, such as cheese, fish, meat, vegetables, or salads) or as seasoning products, such as condiments.
Especially, the compositions of the present invention come as liquid nutritional embodiments for oral or enteral administration that comprise one or more nutrients such as fats, carbohydrates, proteins, vitamins, and minerals. Oral liquid nutritionals are preferred for applications as medical, clinical, or dietetic nutritional products, in particular as beverage, for example a flavored beverage.
These nutritional liquids are preferably formulated with sufficient viscosity, flow, or other physical or chemical characteristics to provide a more effective and soothing coating of the affected mucosa while drinking or administering the nutritional liquid. These nutritional embodiments also preferably represent a balanced nutritional source suitable for meeting the sole, primary, or supplemental nutrition needs of the individual.

Non-limiting examples of suitable nutritional liquids within which the extracts can be formulated, and thus form selected nutritional liquid embodiments of the present invention, are dairy products such as milk and yoghurt; soy based 'dairy-like' products or a flavoured beverage.

Proteins suitable for use herein can be hydrolyzed, partially hydrolyzed or non-hydrolyzed, and can be derived from any known or otherwise suitable source such as milk (e.g. casein, whey), animal (e.g. meat, fish), cereal (e.g. rice, corn), vegetable (e.g. soy), or combinations thereof.

Fats or lipids suitable for use in the nutritional or food supplements include, but are not limited to, coconut oil, soy oil, corn oil, olive oil, safflower oil, high oleic safflower oil, MCT oil (medium chain triglycerides), sunflower oil, high oleic sunflower oil, structured triglycerides, palm and palm kernel oils, palm olein, canola oil, marine oils, cottonseed oils, and combinations thereof.

Carbohydrates suitable for use in the nutritional or food supplements may be simple or complex, lactose- containing or lactose-free, or combinations thereof. Non-limiting examples of suitable carbohydrates include hydrolyzed corn starch, maltodextrin, glucose polymers, sucrose, corn syrup, corn syrup solids, rice-derived carbohydrate, glucose, fructose, lactose, high fructose corn syrup and indigestible oligosaccharides such as fructo-oligosaccharides (FOS), and combinations thereof.

The nutritional or food supplements or feed supplements may further comprise any of a variety of vitamins, non-limiting examples of which include vitamin A, vitamin D, vitamin E, vitamin K, thiamine, riboflavin, pyridoxine, vitamin B12, niacin, folic acid, pantothenic acid, biotin, vitamin C, choline, inositol, salts and derivatives thereof, and combinations thereof.

A nutritional or food supplement or feed supplement according to the present invention may further comprise minerals and trace elements such as sodium, potassium, phosphorus, magnesium, copper, zinc, iron, selenium, chromium and molybdenum.

### EXAMPLES

### The effect of dietary fibers on antibiotic resistant bacteria in the gut microbiota of older adults self-reporting gastrointestinal symptoms

### METHODOLOGY

### Study design

The present study is a parallel controlled trial comprised of 3 groups of 20 elderly people each (≥ 65 years) and randomized to take 12g of either of the prebiotic fibres, an arabinoxylan (=AX) composition comprising more than 30% of arabinoxylans from wheat with an average degree of polymerization of about 52 and an average degree of substitution of about 0.7, Oat β-glucan or placebo (maltodextrin) daily for 6 weeks (42 days). The target population was older individuals, representing the general population. Participants were recruited from already established cohorts, as well as through advertisements in local and regional newspapers. One week prior to the start of the prebiotics supplementation regime, participants were instructed on how to collect fecal samples at home, as outlined in figure 1. They were also asked to answer the questionnaire, "gastrointestinal symptoms rating scale" (GSRS) for self-evaluation of their gut health and experience of gastrointestinal symptoms before and after intervention.

### Recruitment of study participants

An information letter, together with an informed consent form, was sent to all the established cohorts of elderly persons of 65 years and older that indicated an interest in participating in research studies. In addition, recruitment was also performed through pensioner's associations. Study participants returning the consent form were enrolled in the inclusion/exclusion screening. Ethical approval was obtained from the Uppsala regional Ethics review board in Sweden (dnr: 2015/357).

### Collection of demographic data

The following demographic data was collected:
- Age
- Sex
- Country of birth
- Number of older and younger siblings
- Number of biological children
- Marital status
- Education
- Retirement year and age
- Latest profession for subject and partner according to Swedish standard classification of professions (SSYK) by Statistiska Central Byrån (SCB)
- Concomitant diseases

### Medication:

Medication prescriptions, including antibiotics and motility regulating substances, were documented in the case report form (CRF), as well as a study diary provided to the participants at study start. In addition, the subjects were asked if they were currently taking, or had taken additional probiotic substances during the past 6 month, including motility regulating substances purchased over the counter.

### Evaluation of gut health through Gastroinstestinal symptoms rating scale (GSRS)

Gastrointestinal symptoms were assessed through the GSRS clinical rating scale. The reliability and validity of the GSRS is well documented (Svedlund et al., 1988) and norm values for the general population are available (Dimenäs et al., 1995, 1996) from several countries (Kulich et al., 2008). The GSRS includes 15 symptoms divided into 5 domains; reflux, abdominal pain, indigestion, diarrhoea and constipation. The scale is a 7-point Likert scale in which 1 represents no symptoms and 7 represents severe Gl symptoms. According to previous analyses of elderly persons (Östlund-Lagerström et al., 2015, 2016), moderate symptoms were graded as a score ≥ 3. In this study the GSRS was used to monitor gastrointestinal symptoms before, during and after intervention.

### Collection of fecal samples and extraction of microbial DNA

All participants were asked to avoid any use of probiotic compounds one-month prior to the collection of the stool samples. Material for the sample collection was sent via mail. The samples collected were immediately stored at -20°C and transferred to the University lab within one week where they were stored at -80°C until DNA extraction. For analysis of the microbial composition, total DNA was extracted from the fecal samples using the QIAamp DNA stool mini kit according to the manufacturer's instructions (Qiagen) coupled with an initial bead-beating step (Salonen et al., 2010). The bead beating DNA extraction method allows for an unbiased reproducible breaking of all microbial cell walls for complete extraction of microbial DNA.

### Analysis

### Gastrointestinal symptoms rating scale (GSRS)

Twenty-one participants enrolled in the study were identified to suffer from moderate constipation and/or diarrhoea as assessed by a score ≥3 on the GSRS domains, measuring constipation and diarrhoea, respectively. Of these, 13 subjects (4= AX composition described above, 4=Oat-well, 5=placebo) were chosen for further investigation to create an equal number of participants in each intervention group as well as an equal number of females and males.

### Antibiotic resistance markers

The presence of antibiotic resistance markers was evaluated in the participants before and after the intervention using the Qiagen microbial DNA qPCR array for antibiotic resistance genes (Cat. no. 330261 BAID-1901ZRA). The array included 84 resistance genes divided into the following groups: aminoglycoside resistance (6), β-lactamases from Class A (22), Class B (9), Class C (11) and Class D (13), fluoroquinolone resistance (10), macrolide-lincosamide-streptogramin resistance (6), efflux pumps (4), vancomycin resistance (2) and *Staphylococcus aureus* β-lactam resistance gene, *mecA* (1). The qPCR array was performed according to manufactures recommendations. Briefly, 15 ng of genomic DNA together with the master mix was added to each well and the PCR was performed on a MX3000P (Strategene) qPCR machine with cycling conditions of 10 min initial activation at 95°C and 40 cycles of 15 sec denaturation at 95°C and 2 min annealing and extension at 60°C. The threshold was set to 0.1 as recommended and the Ct values were used to determine if the antibiotic resistance gene was detected (+), inconclusive (+/-) or not detected (-). The inconclusive results were further analyzed by agarose gel electrophoresis to confirm the presence of the gene product. The antibiotic resistance gene was considered to be present in the sample if one clear band of appropriate size was seen.

### Statistical analysis

The method for statistical analysis of antibiotic resistance genes was Fisher's test on a 2x3 contingency table for each treatment (AX-COMPOSITION and Oat-well). The contingency table consisted of placebo and one treatment (either AX-COMPOSITION or Oat-well) as rows, as well as less, equal or more resistance genes as columns. For the comparisons of GSRS score changes, a Wilcoxon signed-rank test was used to test whether any significant changes occurred in any treatment group when comparing before and after treatment. For the analysis both R version 3.3.0 and GraphPad Prism 7.0 (GraphPad Software Inc., La Jolla California USA) was used.

### Results:

Intervention with AX-COMPOSITION (n=4) resulted in either equal or lower numbers of antibiotic resistance markers, while the placebo (n=5) or Oat β-glucan (n=4) supplement resulted in equal or higher numbers of antibiotic resistance genes. Difference between AX-COMPOSITION and Placebo were significant (Fisher's exact test, p=0.048)) but no difference was observed between Oat β-glucan and Placebo, see table 1.

**Table 1: Changes of resistance genes in the different groups**

| | Less Resistance genes (-) | Equal number of Resistance genes (+/-) | More resistance genes (+) |
|---|---|---|---|
| Participant group | | AX-COMPOSITION | Oat (β-glucan |
| | AX-COMPOSITION | AX-COMPOSITION | Oat (β-glucan |
| | AX-COMPOSITION | Placebo | Oat (β-glucan |
| | | | Placebo |
| | | | Placebo |
| | | | Placebo |
| | | | Placebo |
| Proportions* : | AX-COMPOSITION - 100% | AX-COMPOSITION - 40% | Placebo - 66% |
| | | Oat (β-glucan - 40% | Oat (β-glucan - 33% |
| | | Placebo - 20% | |
| Total number of participants | 2 | 5 | 6 |

| | | | |
|---|---|---|---|
| **Proportions were significantly different between AX-COMPOSITION and Placebo, p=0.048 (Fischer's exact test)* | | | |

We also observed that AX-COMPOSITION appeared to decrease constipation and diarrhoea among older adults as assessed by GSRS, although the differences between the baseline and intervention were not statistically significant, figure 1 and 2. However, this is a small pilot study that could potentially explain the lack of significance.

### Conclusion:

In conclusion, the results in this pilot study suggest that the presence of increased antibiotic resistance markers in older adults is positively influenced by the AX-composition of the invention and that this is an effective therapeutic strategy to improve overall gut health. Further, the observed results suggest that the reduction in gastro-intestinal complaints is caused by the presence of increased antibiotic resistance markers.

## Claims

1. A composition comprising at least 30% (w/w) of wheat-derived arabinoxylans with an average degree of polymerisation of at least 50 for use in reducing the presence and/or activity of antibiotic resistant bacteria in a subject in need thereof.

2. The composition for use according to claim 1, wherein the composition comprises wheat-derived arabinoxylans with an average degree of polymerisation between 50 and 150 and an average degree of substitution between 0.6 and 0.8.

3. The composition for use according to claim 1 or 2, wherein the composition comprises a protein content of up to 25% (w/w).

4. The composition for use according to anyone of the preceding claims wherein the composition comprises at least 50% (w/w) wheat-derived arabinoxylans.

5. The composition for use according to anyone of the preceding claims wherein at least 30% (w/w) of the composition are water-soluble arabinoxylans.

6. A pharmaceutical composition for use in reducing the presence and/or activity of antibiotic resistant bacteria in a subject in need thereof, comprising a composition for use according to anyone of the preceding claims and at least one acceptable pharmaceutical carrier.

7. A food or feed supplement for use in reducing the presence and/or activity of antibiotic resistant bacteria in a subject in need thereof, comprising a composition for use according to anyone of claims 1 to 5.

8. A combination of at least two compositions for use in reducing the presence and/or activity of antibiotic resistant bacteria in a subject in need thereof, the first composition being a composition or supplement as defined in any one of claims 1 to 7, and the second composition comprising a probiotic.

9. The combination of claim 8 wherein the probiotic is a Bifidobacterium.

10. A combination of at least two compositions for use in reducing the presence and/or activity of antibiotic resistant bacteria in a subject in need thereof, the first composition being a composition or supplement as defined in any one of claims 1 to 7, and the second composition comprising an antibioticum.

11. The combination of at least two compositions for use according to anyone of claims 8 to 10 wherein the at least two compositions are administered simultaneously.

12. The combination of at least two compositions for use according to anyone of claims 8 to 10 wherein the at least two compositions are administered separately.

13. The composition for use according to anyone of claims 1 to 5, or the pharmaceutical composition for use according to claim 6, or the food or feed supplement for use according to claim 7, or the combination of at least two composition for use according to anyone of claims 8 to 12 wherein the subject is a mammal or a bird.

14. The composition for use according to anyone of claims 1 to 5, or the pharmaceutical composition for use according to claim 6, or the food or feed supplement for use according to claim 7, or the combination of at least two composition for use according to anyone of claims 8 to 12 for use in reducing the presence of antibiotic resistant bacteria in the gastrointestinal tract of a mammal.

15. The composition for use according to anyone of claims 1 to 5, or the pharmaceutical composition for use according to claim 6, or the food or feed supplement for use according to claim 7, or the combination of at least two composition for use according to anyone of claims 8 to 12 for use in reducing the expression of antibiotic resistant genes in the gastrointestinal tract of a mammal.
